# EUROPEAN PATENT SPECIFICATION

(11) **EP 0 938 284 B1**
(45) Date of publication and mention of the grant of the patent: **25.09.2002**
(21) Application number: 96937852.0
(22) Date of filing: 04.11.1996
(51) Int. Cl.: A61K 7/06

(54) **HAIR STYLING COMPOSITION**
HAARFORMUNGSMITTEL
COMPOSITION DE MAINTIEN D'UNE COIFFURE

(43) Date of publication of application: 01.09.1999
(73) Proprietor: THE PROCTER & GAMBLE COMPANY, Cincinnati, Ohio 45202 (US)
(72) Inventor: NAMBU, Takanori, Kobe-City, Hyogo 657 (JP)
(74) Representative: Kohol, Sonia
(86) International application number: US9617519
(87) International publication number: WO98019653

(56) References cited:
- EP-A- 0 470 381
- EP-A- 0 685 219
- GB-A- 2 123 694
- GB-A- 2 134 784
- GB-A- 2 136 689

## Description

### TECHNICAL FIELD

The present invention relates to a hair styling composition which provides flexible and good holding film texture.

### BACKGROUND

The desire to have the hair retain a particular shape is widely held. The most common methodology for accomplishing this is the application of a composition to dampened hair, after shampooing and/or conditioning, or to dry, styled hair. These styling compositions provide temporary setting benefits and they can be removed by water or by shampooing. The materials used in the compositions to provide the setting benefits have generally been resins and have been applied in the form of mousses, gels, lotions or sprays. Furthermore, these styling compositions are formulated for additional purposes such as shampooing, conditioning, treating, dyeing, and combinations thereof.

A major desire by the consumer regarding performance of styling compositions has been the ability to hold the hair. Good hold has often been associated with strong and long styling and/or setting of the hair. However, it was also noticed that styling compositions having holding ability beyond a certain degree can also leave the hair feel rough, difficult to comb out, and can be difficult to remove upon shampooing. Further, in recent years, some consumers have expressed a desire to have hair styling products which can allow re-arrangement of hair without compromise to hair holding ability. As styling compositions with good holding ability commonly provide a rigid film texture, it was difficult to provide a composition with flexible film texture which would allow re-arrangement of hair.

Thus, there is a desire to have hair styling compositions which have a good hold without compromise to ability to re-arrange hair, can provide supple, soft feeling to the hair, and can be removed easily from the hair upon shampooing.

It has been known in the art that mixing different type of polymers can result in a composition alleviating the disadvantages of the specific polymer. For example, anionic polymers can give good hold, however, also may provide a rough feel to the hair and are relatively difficult to remove upon shampooing. This is because anionic polymers are generally less hydrophilic after film forming, and tend to repulse shampoo compositions which usually have anionic charges. Cationic polymers can provide supple and soft feel to the hair and can also control static generated by combing etc., however, are also relatively difficult to remove upon shampooing. This is because they tend to deposit on hair which is negatively charged and, over time, may accumulate on hair so that it cannot be easily removed by shampooing. Amphoteric polymers generally have better removability upon shampooing than anionic polymers, and have less deposition/accumulation problems than cationic polymers. However, when used alone, it has limited formulation compatibility to provide a flexible and good holding film texture. Japanese Patent Laid-open 2-223509 discloses a soap-free water-soluble hair styling composition comprising a polymer with both cationic and amphoteric units and an anionic polymer at a certain weight ratio.

In the present invention, a hair styling composition comprising three different types of polymers, anionic polymer, cationic polymer, and amphoteric polymer; at certain levels and ratios with an appropriate solvent have been developed which provides flexible and good holding film texture, which cannot be achieved by single use of or combination of two of any of the above mentioned three types of polymers.

### SUMMARY

The present invention relates to a hair styling composition comprising by weight:
(a) from 0.2% to 5% of an anionic polymer;
(b) from 0.2% to 5% of a cationic polymer;
(c) from 0.2% to 5% of an amphoteric polymer; and
(d) a solvent;
wherein the total of components (a), (b), and (c) is from 0.6% to about 15%, and wherein the level of the component comprised at the lowest level among components (a), (b), and (c) is at least 5% of that of the component comprised at the highest level among components (a), (b), and (c).

Such compositions satisfy the need for a hair styling composition which provides flexible and good holding film texture, thereby providing ability of re-arrangement of the hair, supple and soft feeling of the hair, and good removal upon shampooing, without compromise to good holding ability of the hair. Such compositions further satisfy the general requirement of a styling composition which can comprise a wide range of solvents, preservatives, and propellants.

### DETAILED DESCRIPTION

All percentages herein are by weight of the compositions unless otherwise indicated. All ratios are weight ratios unless otherwise indicated. The total of components except for propellant is hereinafter defined as a "concentrate". For non-aerosol products containing no propellant, the concentrate is equal to the entire composition. All percentages, ratios, and levels of ingredients referred to herein are based on the actual amount of the ingredient, and do not include solvents, fillers, or other materials with which the ingredient may be combined as commercially available products, unless otherwise indicated.

The invention hereof can comprise, consist of, or consist essentially of the essential elements described herein as well as any of the preferred or optional ingredients also described herein.

All publications, patent applications, and issued patents mentioned hereinbelow are hereby incorporated in their entirety by reference.

### AMPHOTERIC POLYMER

Amphoteric polymers are comprised at a level of from 0.2% to 5% by weight of the concentrate.

The amphoteric polymers useful for the present invention are chosen from amongst the following polymers (1) to (5).
(1) Useful herein are polymers of betainised dialkylaminoalkyl (meth)acrylate or dialkylaminoalkyl (meth)acrylamide containing at least units of the formula: 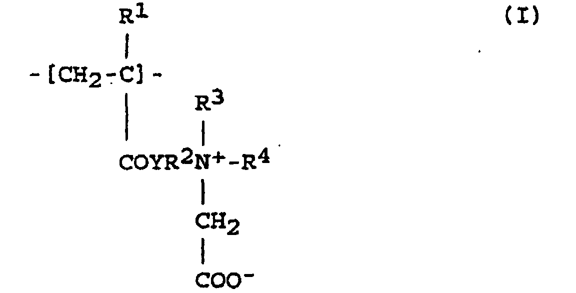 wherein R¹ denotes a hydrogen atom or a methyl group, R² denotes an alkylene group having 1 to 4 carbon atoms, Y denotes O or -NH- and R³ and R⁴ independently of one another denote hydrogen or alkyl having 1 to 4 carbon atoms, and one cationic derivative consisting of a cationic surfactant containing at least one nitrogen atom joined to one or more fatty chains and optionally quaternised, or consisting of a cationic polymer of the polyamine, polyaminopolyamide or poly-(quaternary ammonium) type, the amine or ammonium groups forming part of the polymer chain or being joined thereto. These polymers usually have a molecular weight of 500 to 2,000,000.
   The amphoteric polymers containing units corresponding to the above formula (I) are generally in the form of copolymers which contain, in addition to the units of the above mentioned formula (I), at least units of the formula: 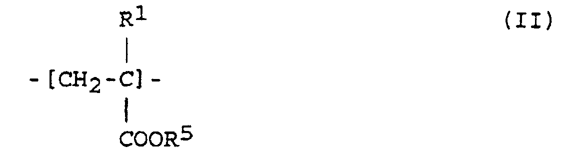 wherein R¹ is as defined above and R⁵ represents an alkyl or alkenyl radical having from 4 to 24 carbon atoms or a cycloalkyl radical having from 4 to 24 carbon atoms.
   It is also possible to use terpolymers, tetrapolymers or pentapolymers which contain, in addition to the units (I) and (II) defined above, units of the formula: 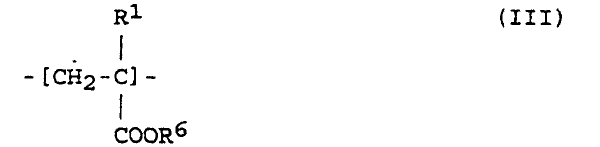 wherein R⁶ preferably denotes an alkyl or alkenyl group having 1 to 3 carbon atoms and R¹ is as defined above.
   The units of the formula (I) are preferably present in an amount of 25 to 45% by weight, units of the formula (II) are preferably present in an amount of 5 to 65% by weight, and units of the formula (III) are preferably present in an amount up to 50% by weight, relative to the total weight of the polymer.
   A particularly preferred polymer is the copolymer containing units of the formulae (I), (II) and (III) in which Y denotes an oxygen atom, R² denotes the group -C₂H₄-, R¹, R³ and R⁴ denote methyl, R⁵ denotes an alkyl group having 4 to 18 carbon atoms and R⁶ denotes an alkyl group having 1 to 3 carbon atoms. The average molecular weight of this polymer is preferably from 70,000 to 90,000. This polymer is sold under the trademark "Yukaformer" or "Diaformer" supplied by Mitsubishi Chemical Corporation.
(2) Useful herein are the polymers resulting from the copolymerisation of a vinyl monomer carrying a carboxyl group, such as acrylic acid, methacrylic acid, maleic acid or alphachloroacrylic acid, and a basic monomer which is a substituted vinyl compound containing at least one basic nitrogen atom, such as dialkylaminoalkyl methacrylates and acrylates and dialkylaminoalkylmethacrylamides and -acrylamides.
(3) Useful herein are the polymers containing units derived from
   i) at least one monomer chosen from amongst acrylamides or methacrylamides substituted on the nitrogen by an alkyl radical,
   ii) at least one acid comonomer containing one or more reactive carboxyl groups, and
   iii) at least one basic comonomer, such as esters, with primary, secondary and tertiary amine substituents and quarternary ammonium substituents, of acrylic and methacrylic acids, and the product resulting from the quaternisation of dimethylaminoethyl methacrylate with dimethyl or diethyl sulphate.

   The N-substituted acrylamides or methacrylamides which are most particularly preferred are the groups in which the alkyl radicals contain from 2 to 12 carbon atoms, especially N-ethylacrylamide, N-tert.-butylacrylamide, N-tert.-octylacrylamide, N-octylacrylamide, N-decylacrylamide and N-dodecylacrylamide and also the corresponding methacrylamides. The acid comonomers are chosen more particularly from amongst acrylic, methacrylic, crotonic, itaconic, maleic and fumaric acids and also the alkyl monoesters of maleic acid or fumaric acid in which alkyl has 1 to 4 carbon atoms.
   The preferred basic comonomers are aminoethyl, butylaminoethyl, N,N'-dimethylaminoethyl and N-tert.-butylaminoethyl methacrylates.
(4) Useful herein are the crosslinked and alkylated polyaminoamides partially or totally derived from polyaminoamides of the general formula:

   -[OC-R-CO-Z]- (I)

   wherein R represents a divalent radical derived from a saturated dicarboxylic acid, from a monocarboxylic or dicarboxylic aliphatic acid with an ethylenic double bond, or from an ester of a lower alkanol having 1 to 6 carbon atoms and of these acids or of a radical derived from the addition of any one of the said acids onto a bis-primary or bis-secondary amine, and Z denotes a radical of a bis-primary or mono- or bis-secondary polyalkylene-polyamine, and preferably represents:
   i) in proportions of 60 to 100 mol %, the radical

      -[NH-(CH₂)ₓ-NH]- (II)

      wherein x is 2 and N is 2 or 3 or alternatively x is 3 and n is 2, this radical being derived from diethylenetriamine, triethylenetetramine or dipropylenetriamine;
   ii) in proportions of 0 to 40 mol %, the above radical (II) wherein x is 2 and n is 1 and which is derived from ethylenediamine, or the radical 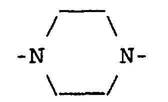 derived from piperazine; and
   iii) in proportions of 0 to 20 mol %, the radical -NH-(CH₂)₆-NH-, derived from hexamethylenediamine, these polyaminoamides being crosslinked by the addition of a difunctional crosslinking agent chosen from amongst epihalogenohydrins, diepoxides, dianhydrides, and bis-unsaturated derivatives, using 0.025 to 0.35 mol of crosslinking agent per amine group of the polyaminoamide, and being alkylated by reaction with acrylic acid, chloroacetic acid or an alkane-sultone or their salts.

   The saturated carboxylic acids are preferably chosen from amongst acids having 6 to 10 carbon atoms, such as adipic acid, 2,2,4- and 2,4,4-trimethyladipic acids, terephthalic acid and acids with an ethylenic double bond, such as acrylic, methacrylic and itaconic acids.
   The alkane-sultones used in the alkylation are preferably propane- or butane-sultone, and the salts of the alkylating agents are preferably the sodium or potassium salts.
(5) Useful herein are the polymers containing zwitterionic units derived form the formula: 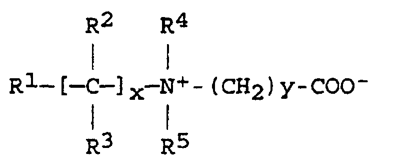 wherein R¹ denotes a polymerisable unsaturated group, such as an acrylate, methacrylate, acrylamide or methacrylamide group, x and y independently represent an integer from 1 to 3, R² and R³ independently represent hydrogen, methyl, ethyl or propyl, and R⁴ and R⁵ independently represent a hydrogen atom or an alkyl radical such that the sum of the carbon atoms in R⁴ and R⁵ does not exceed 10.

Highly preferred amphoteric polymers include commercially available material such as octylacrylamine/acrylates/butylaminoethyl methoacrylate copolymers with the tradenames; AMPHOMER, AMPHOMER LV71, and AMPHOMER LV47 supplied by National Starch & Chemical, and methoacryloyl ethylbetaine/acrylates copolymers with the tradenames; YUKAFORMER SM, YUKAFORMER 301, YUKAFORMER 510, YUKAFORMER M-75, and YUKAFORMER R250S supplied by Mitsubishi Chemical Corporation.

### ANIONIC POLYMER

Anionic polymers are comprised at a level of from about 0.2% to about 5% by weight of the concentrate.

The anionic polymers useful in the present invention are polymers containing units derived from a carboxylic, sulphonic or phosphoric acid and usually have a molecular weight of 500 to 5,000,000. These polymers are water-soluble polymers, it being possible for this solubility to be obtained by neutralisation.

The carboxylic acid groups can be provided by unsaturated monocarboxylic or dicarboxylic acids, such as those corresponding to the formula: 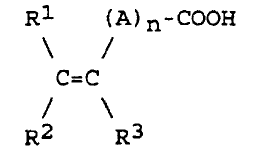 wherein n is 0 or an integer from 1 to 10, A denotes a methylene group optionally joined to the carbon atom of the saturated group, or to the adjacent methylene group in the case where n is greater than 1, via a heteroatom, such as oxygen or sulphur, R¹ denotes a hydrogen atom or a phenyl or benzyl group, R² denotes a hydrogen atom, a lower alkyl group or a carboxyl group and R³ denotes a hydrogen atom, a lower alkyl group, CH₂COOH, or a phenyl or benzyl group.

According to the invention, the preferred anionic polymers containing carboxylic acid groups are:
(1) Hompolymers or copolymers of acrylic or methacrylic acid or salts thereof, and in particular, the products sold under the name VERSICOL E or K by BASF and under the name DARVAN No. 7 by Van der Bilt; acrylic acid/acrylamide copolymers sold in the form of their sodium salt under the name RETEN 421, 423 or 425 by HERCULES; and the sodium salts of polyhydroxycarboxylic acids, sold under the name HYDAGEN F by HENKEL.
(2) Copolymers of acrylic or methacrylic acid with a monoethylenic monomer, such as ethylene, styrene, a vinyl or allyl ester or acrylic or methacrylic acid ester, optionally grafted onto a polyalkylene glycol, such as polyethylene glycol, and optionally corsslinked. Other such copolymers contain an optionally N-alkylated and/or N-hydroxylated acrylamide unit in their chain, such as those sold under the name QUADRAMER 5 by American Cyanamid.
(3) Copolymers derived from crotonic acid, such as those containing, in their chain, vinyl acetate or propionate units and optionally other monomers such as allyl of methallyl esters, a vinyl ether or a vinyl ester of a saturated linear or branched carboxylic acid with a hydrocarbon chain of at least 5 carbon atoms, if appropriate, for these polymers to be grafted and corsslinked, or also a vinyl, allyl or methallyl ester of an α- or β-cyclic carboxylic acid. Included in this class are those with the tradename RESYN 28-2930, 28-2913, and 28-1310 sold by National Starch & Chemicals.
(4) Polymers derived from maleic, fumaric and itaconic acids or anhydrides with vinyl esters, vinyl ethers, vinyl halides, phenylvinyl derivatives, acrylic acid and its esters, such as those sold under the name GANTREZ A, SP, and ES by ISP. Other polymers included in this class are copolymers of maleic, citraconic and itaconic anhydrides with an allyl or methallyl ester optionally containing an acrylamido or methacrylamido group, or with an α-olefine, acrylic or methacrylic acid ester, acrylic or methacrylic acid or vinylpyrrolidone unit in their chain; the anhydride groups can be monoesterified or monoamidified.
(5) Polyacrylamides containing carboxylate groups. Polymers comprising sulphonic groups include polymers containing vinylsulphonic, styrenesulphonic, lignosulphonic or naphthalenesulphonic units. These polymers are chosen, in particular, from amongst:
   i) Polyvinylsulphonic acid salts having a molecular weight of 1,000 to 100,000, and also copolymers with an unsaturated comonomer, such as acrylic or methacrylic acid or an ester thereof and also substituted or unsubstituted acrylamide or methacrylamide, vinyl esters, vinyl ethers and vinylpyrrolidone.
   ii) Polystyrenesulphonic acid salts, such as the sodium salt sold by National Starch & Chemicals under the name Flexan 500 and 130.
   iii) Alkali metal or alkaline earth metal salts of sulphonic acids derived from lignin, and more particularly calcium lignosulphonates or sodium lignosulphonates, such as the product sold under the name Marasperse C-21 by American Can Co. and the C₁₀ to C₁₄ products sold by Avebene.
   iv) Polymers containing salified alkylnaphthalenesulphonic acid units, such as the sodium salt under the name Darvan No. 1 by Van der Bilt.

The anionic polymers herein which include anionic monomers are preferably utilised in at least partially neutralised form in order to aid shampoo removability of the liquid hair cosmetic compositions. In the compositions according to the present invention the neutralisation of a polymer may be achieved by use of an inorganic base, preferably KOH. However organic base, preferably AMP (amino methyl propanol) and mixture of inorganic and organic base may also be used to effect the desired level of neutralisation in hair styling compositions according to the present invention. In total from about 50% to about 100%, preferably from about 70% to about 100%, most preferably from about 80% to about 100% of the acidic monomers of each polymer utilised should be neutralised with base.

Any conventionally used base, organic or inorganic, may be used for neutralisation of acidic polymers provided they are utilised as specified herein. Hydroxides of alkali, alkaline earth and amino alcohols are suitable neutralisers for use in the present invention.

Examples of suitable organic neutralizing agents which may be included in the compositions of the present invention include amines, especially amino alcohols such as 2-amino-2-methyl-1, 3-propanediol (AMPD), 2-amine-2ethyl-1, 3-propanediol (AEPD), 2-amino-2-methyl-1-propanol (AMP), 2-amino-1-butanol (AB), monethanolamine (MEA), diethanolamine (DEA), triethanolamine (TEA), monoisopropanolamine (MIPA), diisopropanolamine (DIPA), triisopropanolamine (TIPA), dimethylsteramine (DMS) and amino methyl propanol (AMP) and mixtures thereof.

Preferred neutralising agents for use in hair styling compositions of the present invention are potassium and sodium hydroxides.

Highly preferred anionic polymers include commercially available material such as vinyl acetate/crotonic acid/vinyl neodecanoate copolymers and vinyl acetate/crotonic acid copolymers with the tradenames RESYN 28-2930, RESYN 28-2913, and RESYN 28-1310 supplied by National Starch & Chemicals, and acrylates copolymers and acrylates/acrylamide copolymers with tradenames LUVIMER 100P, ULTRAHOLD 8, and ULTRAHOLD STRONG supplied by BASF Corporation.

### CATIONIC POLYMER

Cationic polymers are comprised at a level of from about 0.2% to about 5% by weight of the concentrate.

The cationic polymers useful in the present invention are:
(1) Vinylpyrrolidone / quaternized dialkylaminoalkyl acrylate or methacrylate copolymers such as those sold under the tradename Gafquat 734 and 755N by the Gaf Corp.
(2) Cellulose ether derivatives containing quaternary ammonium groups.
(3) Cationic polysaccharides.
(4) Cationic polymers chosen from the group comprising:
   i) polymers containing units of the formula:

      -A-Z¹-A-Z²- (I)

      wherein A denotes a radical containing two amino groups, preferably a piperazinyl radical, and Z¹ and Z² independently denote a divalent radical which is a straight-chain or branched-chain alkylene radical which contains up to about 7 carbon atoms in the main chain, is unsubstituted or substituted by one or more hydroxyl groups and can also contain one or more oxygen, nitrogen and sulphur atoms and 1 to 3 aromatic and/or heterocyclic rings, the oxygen, nitrogen and sulphur atoms generally being present in the form of an ether or thioether, sulphoxide, sulphone, sulphonium, amine, alkylamine, alkenylamine, benzylamine, amine oxide, quaternary ammonium, amide, imide, alcohol, ester and/or urethane group;
   ii) polymers containing units of the formula:

      -A-Z'-A-Z'- (II)

      wherein A denotes a radical containing two amino groups, preferably a piperazinyl radical, and Z' denotes the symbol Z³ and Z⁴ while denoting the symbol Z⁴ at least once; Z³ denotes a divalent radical which is a straight-chain or branched-chain alkylene or hydroxyalkylene radical having up to about 7 carbon atoms in the main chain, and Z⁴ is a divalent radical which is a straight-chain or branched-chain alkylene radical which has up to about 7 carbon atoms in the main chain, is unsubstituted and substituted by one or more hydroxyl radicals and is interrupted by one or more nitrogen atoms, the nitrogen atom being substituted by an alkyl chain having from 1 to 4 carbon atoms, preferably 4 carbon atoms, which is optionally interrupted by an oxygen atom and optionally contains one or more hydroxyl groups; and
   iii) the alkylation products, with alkyl and benzyl halides of 1 to 6 carbon atoms, alkyl tosylates or mesylates, and the oxidation products, of the polymers of the formulae (I) and (II) indicated above under i) and ii).
(5) Polyamino-polyamides prepared by the polycondensation of an acid compound with a polyamine. The acid compound can be organic dicarboxylic acids, aliphatic monocarboxylic and dicarboxylic acids containing a double bond, esters of the abovementioned acids, preferably the esters with lower alkanols having from 1 to 6 carbon atoms, and mixtures thereof. The polyamine is a bis-primary or mono- or bis-secondary polyalkylene-polyamine wherein up to 40 mol% of this polyamine can be a bis-primary amine, preferably ethylenediamine, or a bis-secondary amine, preferably piperazine, and up to 20 mol% can be hexamethylenediamine.
(6) The above mentioned polyamino-polyamides can be alkylated and/or crosslinked. The alkylation can be carried out with glycidol, ethylene oxide, propylene oxide or acrylamide. The crosslinking is carried out by means of a crosslinking agent such as:
   i) epihalogenohydrins, diepoxides, dianhydrides, unsaturated anhydrides and bis-saturated derivatives, in proportions of 0.025 to 0.35 mol of crosslinking agent per amine group of the polyamino-polyamide;
   ii) bis-halogenohydrins, bis-azetidinium compounds, bishalogeno acyldiamines and bis-(alkyl halides);
   iii) oligomers obtained by reacting a compound chosen from the group comprising bis-halogenohydrins, bis-azetidinium compounds, bis-halogenoacyldiamines, bis-(alkyl halides), epihalogenohydrins, diepoxides and bis-unsaturated derivatives, with another compound which is a difunctional compound which is reactive towards the compound; and
   iv) the quaternisation product of a compound chosen from the compounds ii) and the oligomers iii) and containing one or more tertiary amine groups which can be totally or partially alkytated with an alkylating agent preferably chosen from methyl or ethyl chlorides, bromides, iodides, sulphates, mesylates and tosylates, benzyl chloride or bromide, ethylene oxide, propylene oxide and glycidol, the crosslinking being carried out by means of 0.025 to 0.35 mol, in particular of 0.025 to 0.2 mol and more particularly of 0.025 to 0.1 mol, of crosslinking agent per amine group of the polyamino-polyamide.
(7) Polyamino-polyamide derivatives resulting from the condensation of a polyalkylene-polyamine with a polycarboxylic acid, followed by alkylation by means of difunctional agents, such as the adipic acid/dialkylaminohydroxyalkyldialkylenetriamine copolymers in which the alkyl radical contains 1 to 4 carbon atoms and preferably denotes methyl, ethyl or propyl.
   Useful polymers are adipic acid/dimethylaminohydroxypropyldiethylenetriamine copolymers sold under the name Cartaretine F, F⁴ or F⁸ by SANDOZ.
(8) Polymers obtained by reacting polyalkylenepolyamine containing two primary amine groups and at least one secondary amine group, with a dicarboxylic acid chosen from diglycolic acid and saturated aliphatic dicarboxylic acids having 3 to 8 carbon atoms, the molar ratio of the polyalkylene-polyamine to the dicarboxylic acid being from 0.8:1 to 1.4:1, and the resulting polyamide being reacted with epichlorohydrin in a molar ratio of epichlorohydrin to the secondary amine groups of the polyamide of from 0.5:1 to 1.8:1.
   Useful polymers are those sold under the name HERCOSETT 57 by Hercules Incorporated, and that sold under the name PD 170 or DELSETTE 101 by Hercules.
(9) Cyclic polymers generally having a molecular weight of 20,000 to 3,000,000 such as homopolymers containing, as the main constituent of the chain, units corresponding to the formula (III) or (III') 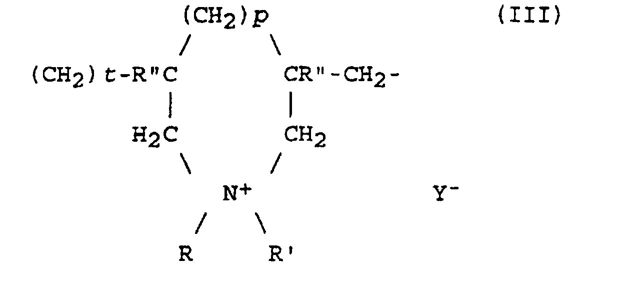 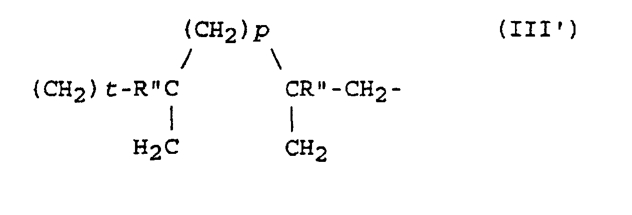 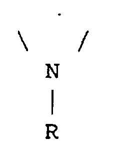 in which *p* and *t* are 0 or 1, and *p*+*t*=1, R" denotes hydrogen or methyl, R and R' independently of one another denote an alkyl group having from 1 to 22 carbon-atoms, a hydroxylalkyl group in which the alkyl group preferably has 1 to 5 carbon atoms, or a lower amidoalkyl group, and R and R' can denote, together with the nitrogen atom to which they are attached, heterocyclic groups such as piperidinyl or morpholinyl, and Y is bromide, chloride, acetate, borate, citrate, tartrate, bisulphate, bisulphite, sulphate or phosphate. Copolymers containing units of the formula III and III' may also contain units derived from acrylamide or from diacetoneacrylamide.
   Amongst the quaternary ammonium polymers of the type defined above, those which are preferred are the dimethyldiallylammonium chloride homopolymer sold under the name MERQUAT 100 and having a molecular weight of less than 100,000, and the dimethyldiallylammonium chloride/acrylamide copolymer having a molecular weight of more than 500,000 and sold under the name MERQUAT 550 by CALGON Corporation.
(10) Poly-(quaternary ammonium) compounds of the formula 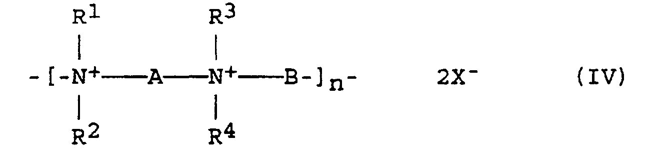 wherein R¹, R², R³, and R⁴ are independently aliphatic, alicyclic or arylaliphatic radicals containing a maximum of 20 carbon atoms, or lower hydroxyaliphatic radicals, or alternatively, with the nitrogen atoms to which they are attached, heterocyclic rings optionally containing a second hetero-atom other than nitrogen, or alternatively R¹, R², R³, and R⁴ represent a group CH₂CHR'³R'⁴ wherein R'³ denoting hydrogen or lower alkyl and R'⁴ denoting SO, CN, CON(R'⁶)₂, COOR'⁵, COR'⁵, COOR'⁷D, or CONHR'⁷D; R'⁵ denoting lower alkyl, R'⁶ denoting hydrogen or lower alkyl, R'⁷ denoting alkylene and D denoting a quaternary ammonium group; A and B independently represent a polymethylene group containing from 2 to 20 carbon atoms, which can be linear or branched, saturated or unsaturated and can contain, inserted in the main chain one or more groups -CH₂-Y-CH₂- wherein Y denotes benzene, oxygen, sulfur, SO, SO₂, SS, NR'⁸, N+(R'⁹)₂X¹⁻, CHOH, NHCONH, CONR'⁸, or COO; X¹⁻ denoting an anion derived from a mineral or organic acid, R'⁸ denoting hydrogen or lower alkyl and R'⁹ denoting lower alkyl, or alternatively A and R¹ and R³ form a piperazine ring with the two nitrogen atoms to which they are attached. If A denotes a linear or branched, saturated or unsaturated alkylene or hydroxyalkylene radical, B can also denote a group: -(CH₂)ₙ-CO-D-OC-(CH₂)ₙ-; wherein n is selected so that the molecular weight is generally between 1,000 and 100,000; and D denotes:
   i) a glycol radical of the formula -O-Z-O-, in which Z denotes a linear or branched hydrocarbon radical or a group corresponding to the fomulae:
      -[CH₂-CH₂-O-]_{*x*}-CH₂-CH₂- or -[CH₂-C(CH₃)H-O-]_{*y*}-CH₂-C(CH₃)H- wherein x and y denote an integer from 1 to 4, representing a definite and unique degree of polymerisation;
   ii) a bis-secondary diamine radical, such as a piperazine derivative;
   iii) a bis-primary diamine radical of the formula: -N-H-Y-NH-, in which Y denotes a linear or branched hydrocarbon radical or the divalent radical -CH₂-CH₂-S-S-CH₂-CH₂-; or
   iv) a ureylene group of the formula -N-H-CO-NH-.
(11) Homopolymers or copolymers derived from acrylic or methacrylic acid and containing at least one unit: 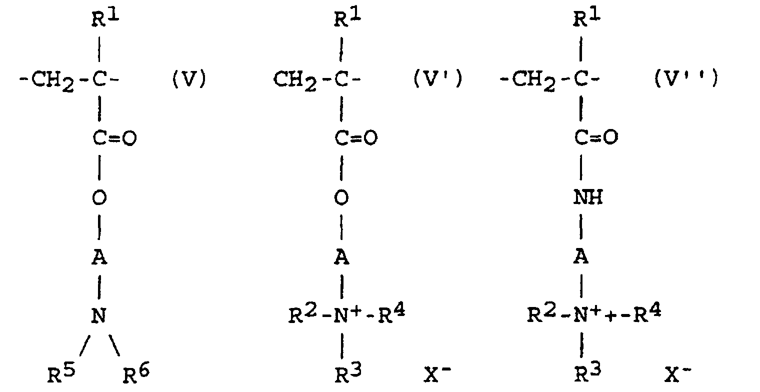 wherein R¹ is H or CH₃, A is a linear or branched alkyl group having 1 to 6 carbon atoms or a hydroxyalkyl group having 1 to 4 carbon atoms, R², R³ and R⁴ independently denote an alkyl group having 1 to 18 carbon atoms or a benzyl group, R⁵ and R⁶ denote H or alkyl having 1 to 6 carbon atoms and X denotes methosulphate or halide, such as chloride or bromide.
   The comonomer or comonomers which can be used typically belong to the family comprising: acrylamide, methacrylamide, diacetone-acrylamide, acrylamide and methacrylamide substituted on the nitrogen by one or more lower alkyls, alkyl esters of acrylic and methacrylic acids, vinylpyrrolidone and vinyl esters.
   Useful polymers are Quatemium 38, 37, 49 and 42 in the CTFA, acrylamide/beta-methacryloyloxyethyl-trime-thylammonium methosulphate copolymers sold under the names Teten 205,210,220 and 240 by Hercules, and aminoethylacrylate phosphate/acrylate copolymer sold under the name Catrex by National Starch & Chemicals, and the crosslinked graft cationic copolymers having a molecular weight of 10,000 to 1,000,000, and preferably of 15,000 to 500,000, and resulting from the copolymerisation of: at least one cosmetic monomer, dimethylaminoethyl methacrylate, polyethylene glycol and a polyunsaturated crosslinking agent, such as those mentioned in the CTFA dictionary under the name AMODIMETHICONE, such as the product marketed as a mixture with other ingredients under the name DOW CORNING 929 cationic emulsion.
(12) Other cationic polymers which can be used are polyalkyleneimines, in particular polyethyleneimines, polymers containing vinylpyridine units or vinylpyridinium units in the chain, condensates of polyamines and of epichlorohydrin, poly-(quaternary ureylenes) and chitin derivatives.

Highly preferred cationic polymers include commercially available material such as Polyquaternium 4 under the tradenames CELQUAT H100 and CELQUAT L200 supplied by National Starch & Chemicals, and Polyquaternium 11 under the tradename GAFQUAT 755N supplied by ISP.

### SOLVENT

Solvents used in the present invention are selected depending on variables such as the remainder of components, viscosity, and desired foaming characteristic of the composition.

Non-limiting examples of solvents useful in the present invention are: water, lower alcohols having 1 to 6 carbons such as ethanol and isopropanol, and polyhydric alcohols such as propylene glycol, hexylene glycol, glycerin, and propane diol, and mixtures thereof. For mousse compositions, the solvent comprises at least 80% water, more preferably at least 90% water.

The solvent is preferably comprised at a level by weight of from 60% to 99%, more preferably from 80 to 99%, most preferably from 85% to 98% of the concentrate.

### NONIONIC SURFACTANTS

When the styling composition of the present invention is in the form of a mousse, the composition preferably comprises a nonionic surfactant which has a total HLB value by Griffin method of at least 12. These nonionic surfactants are particularly preferred as they provide softness to the foam.

In the present invention, the HLB value is measured by Griffin method as follows:
For polyhydric alcohols and fatty acid esters, the HLB value is calculated by the following calculated general formula:

   HLB=20(1-S/A)

   wherein S is saponification value of esters, and A is the acid value of fatty acids;
For surfactants of which the saponification value is not clear, the HLB value is calculated by the following formula:

   HLB=(E+P)/5

   wherein E is weight percentage of ethylene oxide, and P is weight percentage of polyhydric alcohol; and
For surfactants wherein polyoxyethylene is the only hydrophilic group, the HLB value is calculated by the following general formula:

   HLB=E/5
wherein E is weight percentage of ethylene oxide.

The styling composition of the present invention preferably comprises a nonionic surfactant selected from the group consisting of a polyxyethylene, a polyoxy propylene, and mixtures thereof which has a total HLB value by Griffin method of at least 12. Another embodiment of the styling composition of the present invention may comprise a first nonionic surfactant selected from the group consisting of the below identified general formula (I), (II), (III) and mixtures thereof, and a second nonionic surfactant selected from the group consisting of the below identified general formula (I), (II), (III), (IV), (V), (VI) and mixtures thereof.

Non-limiting examples of nonionic surfactants for use in the styling composition of the present invention are as follows:
(1) Polyoxyethylene alkyl ether which are polyethylene glycol ethers of alkyl alcohol having the following general structure;

   CH₃ - (CH₂)ₐ- (OCH₂CH₂)ₙ- OH (I)

   wherein a has an average value from 9 to 21, n has an average value from 2 to 200;
(2) Polyoxyethylene alkenyl ether having the following general structure;

   CH₃ - (CH₂)_{b} - CH = CH (CH₂)_{c} - (OCH₂CH₂)ₙ- OH (II)

   wherein b has an average value from 1 to 10, c has an average value from 1 to 10, n has an average value from 2 to 200;
(3) Polyoxypropylene polyoxyethylene alkyl or alkenyl or iso-alkyl or iso-alkenyl or dimethylpolysiloxane ether having the following general structure;

   R¹ - [OCH (CH₃) CH₂]ₓ- (OCH₂CH₂)_{y} - OH (III)

   wherein R¹ is selected from the group consisting of alkyl, alkenyl, iso-alkyl alkenyl, and dimethylpolysiloxane derivatives, x has an average value from 2 to 100; and y has an average value from 2 to 100;
(4) Polyoxyethylene long chain alkyl fatty acid or benzene derivative ether having the following general structure;

   R² - (OCH₂CH₂)ₙ -OH (IV)

   wherein R² is selected from the group consisting of castor oil triglyceride castorate, cholesterol, coconut oil triglyceride cocoate, alkyl phenol, glyceryl laurate, glyceryl oleate, glyceryl cocoate, glyceryl isostearate, glyceryl stearate, hydrogenated castor oil triglyceride hydrogenated castorate, hydrogenated lanolin, nonyl phenyl and dimethylpolysiloxane derivatives, and n has an average value from 2 to 200;
(5) Polyoxyethylene alkyl, or alkenyl ether having the following general structure;

   R³ - C (O) - (OCH₂CH₂)ₙ - O - C(O) -R⁴ (V)

   wherein R³ and R⁴ are independently selected from the group consisting of alkyl, iso-alkyl, and alkenyl, and n has an average value from 2 to 200;
(6) Polyoxypropylene alkyl, iso-alkenyl or long chain alkyl fatty acid ether having the following general structure;

   R⁵- [OCH (CH₃) CH₂]ₙ OH (VI)
wherein R⁵ is selected from the group consisting of alkyl, iso-alkyl and alkenyl, and n has an average value of 2 to 200.

### OPTIONAL COMPONENTS

Optional components can be included in the hair styling compositions of the present invention, depending on the needs of the product. Non-limiting examples of such optional components include conditioning agents, preservatives, perfume, ultraviolet and infrared screening and absorbing agents, colorants, pH adjusters, additional nonionic polymers, additional surfactants, dyes, vitamins, proteins, plant extracts, and nutrients.

### Conditioning agents

Conditioning agents may be comprised in the hair styling composition of the present invention. Suitable conditioning agents include fatty alcohols, fatty acids, and hydrocarbons.

The fatty alcohols useful herein are those having from 10 to 30 carbon atoms, preferably from 12 to 22 carbon atoms, and more preferably from 16 to 22 carbon atoms. These fatty alcohols can be straight or branched chain alcohols and can be saturated or unsaturated. Nonlimiting examples of fatty alcohols include decyl alcohol, undecyl alcohol, dodecyl, myristyl, cetyl alcohol, stearyl alcohol, isostearyl alcohol, isocetyl alcohol, behenyl alcohol, linalool, oleyl alcohol, cholesterol, *cis*-4-*t*-butylcyclohexanol, myricy alcohol and mixtures thereof. Especially preferred fatty alcohols are those selected from the group consisting of cetyl alcohol, stearyl alcohol, isostearyl alcohol, oleyl alcohol, and mixtures thereof. The fatty acids useful herein are those having from 10 to 30 carbon atoms, preferably from 12 to 22 carbon atoms, and more preferably from 16 to 22 carbon atoms. These fatty acids can be straight or branched chain acids and can be saturated or unsaturated. Also included are diacids, triacids, and other multiple acids which meet the carbon number requirement herein. Also included herein are salts of these fatty acids. Nonlimiting examples of fatty acids include lauric acid, palmitic acid, stearic acid, behenic acid, arichidonic acid, oleic acid, isostearic acid, sebacic acid, and mixtures thereof. Especially preferred for use herein are the fatty acids selected from the group consisting of palmitic acid, stearic acid, and mixtures thereof.

Hydrocarbons are useful herein as conditioning agents. Useful hydrocarbons include straight chain, cyclic, and branched chain hydrocarbons which can be either saturated or unsaturated. The hydrocarbons preferably will have from 12 to 40 carbon atoms, more preferably from 12 to 30 carbon atoms, and most preferably from 12 to 22 carbon atoms. Also encompassed herein are polymeric hydrocarbons of alkenyl monomers, such as polymers of C2-C6 alkenyl monomers. These polymers can be straight or branched chain polymers. The straight chain polymers wilt typically be relatively short in length, having a total number of carbon atoms as described above in this paragraph. The branched chain polymers can have substantially higher chain lengths. The number average molecular weight of such materials can vary widely, but will typically be up to about 500, preferably from about 200 to about 400, and more preferably from about 300 to about 350. Also useful herein are the various grades of mineral oils. Mineral oils are liquid mixtures of hydrocarbons that are obtained from petroleum. Specific examples of suitable hydrocarbon materials include paraffin oil, mineral oil, dodecane, isododecane, hexadecane, isohexadecane, eicosene, isoeicosene, tridecane, tetradecane, polybutene, polyisobutene, and mixtures thereof. Isododecane, isohexadeance, and isoeicosene are commercially available as Permethyl 99A, Permethyl 101A, and Permethyl 1082, from Presperse, South Plainfield, NJ. A copolymer of isobutene and normal butene is commercially available as Indopol H-100 from Amoco Chemicals. Preferred for use herein are hydrocarbon conditioning agents selected from the group consisting of mineral oil, isododecane, isohexadecane, polybutene, polyisobutene, and mixtures thereof. When included, these conditioning agents are comprised at a level by weight of from 0.01% to 2% of the concentrate.

### Preservative

Hair styling compositions of the present invention can further comprise a preservative. Such preservative is preferably included at a level by weight of up to 5%, more preferably up to 3% of the concentrate.

Non-limiting examples of preservatives useful in the present invention are DMDM Hydantoin (dimethylol dimethyl hydantoin), Kathon CG, (mixture of methylchloro-isothiazolinone and methyl isothiazolinone), imidazolidinyl urea, phenoxyethanol, EDTA and its salts, benzyl alcohol, and parabens such as methyl paraben, propyl paraben, butyl paraben, and LiquaPar oil (mixture of isobutyl paraben, isopropyl paraben, and butyl paraben).

### Propellant

Propellants may be used for mousse and hair spray compositions. Propellants when used in the present invention are selected depending on variables such as the remainder of components, the package, and whether the product is designed to be used standing or invert.

When comprised in mousses, the propellant is preferably comprised at a level of up to 60%, preferably up to 30% of the entire composition. When comprised in sprays, the propellant is preferably comprised at a level of from 15% to 80%, preferably from 20% to 60% of the entire composition. When no propellant is used, the hair styling composition is usually provided in a package equipped with an air or gas mixing device.

Non-limiting examples of propellants useful in the present invention are: fluorohydrocarbons such as difluoroethane 152a (supplied by DuPont), dimethyl ether, and hydrocarbons such as propane, iso-butane, n-butane, and mixtures of hydrocarbons such as LPG (liquefied petroleum gas).

### Other Optional Components

Hair styling compositions of the present invention may further comprise a variety of optional components. Such optional components include; thickeners and viscosity modifiers such as diethanolamides of long chain fatty acids, sodium chloride, and sodium sulfate, ultraviolet absorbing agents such as octyl salicylate, pH adjusting agents such as citric acid, succinic acid, sodium hydroxide and triethanolamine, coloring agents, hair oxidizing agents such as hydrogen peroxide, perborate salts and persulfate salts, hair reducing agents such as thioglycolates, perfumes, perfume solubilizing agents such as polyethylene glycol fatty acid esters, sequestering agents, polymer plasticizing agents such as glycerin and propylene glycol, and volatile and non-volatile silicone fluids of low molecular weight. Such optional ingredients are typically included at a level by weight of up to about 20%, preferably up to about 10% of the concentrate.

### COMPOSITION

The hair styling composition of the present invention comprise a certain minimum amount of each of an anionic polymer, a cationic polymer, and an amphoteric polymer. The level of the component comprised at the lowest level among these polymers is at least 5% of that of the polymer comprised at the highest level. Without this minimum amount of each polymer, the desired benefits of the present invention cannot be achieved. The total amount of amphoteric polymer, anionic polymer, and cationic polymer by weight of the concentrate is from 0.6% to 15%, preferably from 0.6% to 10%, more preferably from 0.6% to 8%. The amount of each polymer and the ratios to one another is selected by the artisan depending on the characteristic of the desired product.

The three type of polymers are preferably included in the composition in a manner wherein they are easily dispersed and mixed with other components to make a stable final product. It is known in the art that directly mixing anionic polymers and cationic polymers can make precipitations which are difficult to disperse. This is particularly problematic for hair sprays and mousses wherein the final product is applied to the hair as fine mists or foams. A suitable and preferable way of adding the three type of polymers is to add them in an order so that the anionic polymer and the cationic polymer does not directly contact each other.

### EXAMPLES

The following examples further describe and demonstrate embodiments within the scope of the present invention. Ingredients are identified by chemical or CTFA name, or otherwise defined below.

### Method of Preparation

Examples I and II are mousse composition embodiments, and Examples III and IV are hair spray composition embodiments of the present invention which can be prepared by any conventional method well known in the art. a suitable method is as follows:

First, the anionic polymer is neutralized in an aqueous medium. To this is added amphoteric polymer with caution to avoid lumping. Then, remaining ingredients except the cationic polymer are added. Finally, the cationic polymer or an aqueous medium containing the cationic polymer is added, and the obtained mixture is mixed until homogeneous. The concentrate thus obtained is packed into aerosol cans with propellant to make a mousse or spray composition.

| | AMOUNT (%) | | | |
|---|---|---|---|---|
| EXAMPLE NO. | 1 | 2 | 3 | 4 |
| COMPONENTS IN COMPOSITION | | | | |
| Concentrate | 94.0 | 92.0 | 65.0 | 55.0 |
| Propellant L.P.G. | 6.0 | 8.0 | 35.0 | 45.0 |
| Total of Composition | 100 | 100 | 100 | 100 |

| COMPONENTS IN CONCENTRATE | | | | |
|---|---|---|---|---|
| Amphomer LV71 *¹ | 3.50 | - | 4.00 | 3.00 |
| Yukaformer SM *² | - | 10.0 | - | - |
| Luvimer 36D *³ | - | 4.17 | 2.78 | - |
| Resyn 28-2913 *⁴ | 1.00 | - | - | 2.00 |
| Celquat H100 *⁵ | 0.50 | 0.50 | 0.20 | 0.15 |
| Aminomethylpropanol | 0.79 | 0.75 | 1.03 | 0.79 |
| POE (21) lauryl ether | 0.22 | 0.28 | - | - |
| POE (4.2) lauryl ether | 0.18 | 0.23 | - | - |
| Phenyltrimethicone | 0.50 | 0.50 | 0.50 | - |
| Lauramide diethanolamine | 0.10 | 0.10 | - | - |
| Stearyl alcohol | 0.10 | - | - | - |
| Tego Betaine F *⁶ | - | 0.33 | - | - |
| Methyl Paraben | 0.15 | 0.15 | - | - |
| Phenoxyethanol | 0.25 | 0.25 | 0.25 | 0.25 |
| Disodium EDTA | 0.10 | 0.10 | 0.10 | 0.10 |
| Permethyl 99A *⁷ | 0.05 | - | - | 0.10 |
| Propylene glycol | 0.25 | 0.25 | 0.25 | 0.10 |
| Perfume | 0.05 | 0.07 | 0.07 | 0.07 |
| Ethanol 190 proof | - | - | 20.0 | 25.0 |
| DI Water | q.s. | q.s. | q.s. | q.s. |
| Total of Concentrate | 100 | 100 | 100 | 100 |

| | | | | |
|---|---|---|---|---|
| *¹ Amphomer LV71: Octylacrylamide / acrylates / butylaminoethyl / methacrylate copolymer supplied by National Starch & Chemical | | | | |
| *² Yukaformer SM: Methoacryloyl ethyl betaine / acrylates copolymer supplied by Mitsubishi Chemical Corporation | | | | |
| *3 Luvimer 36D: Acrylates copolymer supplied by BASF Corporation | | | | |
| *4 Resyn 2913: Vinyl acetate / crotonic acid / vinylneodecanoate copolymer supplied by National Starch & Chemical | | | | |
| *5 Celquat H100: Polyquaternium 4 supplied by National Starch & Chemical | | | | |
| *6 Tego Betaine F: Cocamidopropyl betaine supplied by Gold Schmidt | | | | |
| *7 Permethyl 99A: Isododecane supplied by Presperse | | | | |

Examples 2 and 4 are not illustrative of the present invention.

## Claims

1. A hair styling composition comprising by weight of the concentrate:
(a) from 0.2% to 5% of an amphoteric polymer;
(b) from 0.2% to 5% of an anionic polymer;
(c) from 0.2% to 5% of a cationic polymer; and
(d) a solvent;
wherein the total of components (a), (b), and (c) is from 0.6 to 15% by weight of the concentrate, and wherein the level of the component comprised at the lowest level among components (a), (b), and (c) is at least 5% of that of the component comprised at the highest level among components (a), (b), and (c).

2. The hair styling composition according to Claim 1 wherein the composition is in the form of a mousse further comprising from 0.02% to 1% by weight of the concentrate of a nonionic surfactant having a total HLB value by Griffin method of at least 12.

3. The hair styling composition according to Claim 1 wherein the composition is in the form of a hair spray.

## Patentansprüche

1. Haarfrisierzusammensetzung, umfassend, bezogen auf Gewicht des Konzentrats:
(a) 0.2% bis 5% eines amphoteren Polymeren:
(b) 0,2% bis 5% eines anionischen Polymeren:
(c) 0,2% bis 5% eines kationischen Polymeren; und
(d) ein Lösungsmittel;
wobei die Gesamtmenge der Komponenten (a), (b) und (c) 0,6 bis 15 Gew.-% des Konzentrats ausmacht, und wobei der Anteil der Komponente, welche unterhalb den Komponenten (a), (b) und (c) im geringsten Anteil vorliegt, mindestens 5% desjenigen der Komponente beträgt, welche unter den Komponenten (a), (b) und (c) mit dem höchsten Anteil vorliegt.

2. Haarfrisierzusammensetzung nach Anspruch 1, wobei die Zusammensetzung in Form eines Schaums vorliegt, umfassend weiterhin 0.02 bis 1 Gew.-% des Konzentrats an einem nichtionischen Tensid mit einem Gesamt-HLB-Wert nach der Griffin-Methode von mindestens 12.

3. Haarfrisierzusammensetzung nach Anspruch 1, wobei die Zusammensetzung in Form eines Haarsprays vorliegt.

## Revendications

1. Composition de stylisation des cheveux comprenant, en poids du concentré :
(a) 0,2% à 5% d'un polymère amphotère ;
(b) 0,2% à 5% d'un polymère anionique ;
(c) 0,2% à 5% d'un polymère cationique ; et
(d) un solvant ;
dans laquelle le total des composants (a), (b), et (c) est 0,6 à 15% en poids du concentré, et dans laquelle la quantité du composant présent en quantité la plus faible parmi les composants (a), (b), et (c) est au moins 5% de celle du composant présent en quantité la plus élevée parmi les composants (a), (b), et (c).

2. Composition de stylisation des cheveux selon la revendication 1, dans laquelle la composition est sous forme d'une mousse comprenant en outre 0,02% à 1% en poids du concentré d'un tensioactif non ionique ayant une HLB totale selon la méthode de Griffin d'au moins 12.

3. Composition de stylisation des cheveux selon la revendication 1, dans laquelle la composition est sous forme d'un spray pour cheveux.
